Europäisches Patentamt

European Patent Office

Office européen des brevets

(11) Publication number: 0 247 552
A2

# EUROPEAN PATENT APPLICATION

(21) Application number: 87107545.3

(22) Date of filing: 23.05.87

(51) Int. Cl.4: B01F 17/42 , A61K 7/00 , A61K 47/00

(30) Priority: 30.05.86 IT 2064386

(43) Date of publication of application:
02.12.87 Bulletin 87/49

(84) Designated Contracting States:
AT BE CH DE ES FR GB GR IT LI LU NL SE

(71) Applicant: SO.RI.FAR. S.r.l.
Via Donizetti 2-2
I-16154 GENOVA(IT)

(72) Inventor: Lusenti, Luciana
Via Donizetti, 2-2
I-16154 Genova(IT)

(74) Representative: Minoja, Fabrizio
Studio Consulenza Brevettuale Via Rossini,
8
I-20122 Milano(IT)

(54) Self-emulsifying fat for pharmaceutical and cosmetic use, preparation and use thereof.

(57) A process for the preparation of a non-ionic self-emulsifying fat having a constant composition, obtained from natural products selected from $C_6$-$C_{18}$ fatty acids, $C_6$-$C_{20}$ polyalcohols and fatty alcohols, esterified and ethoxylated.

The obtained product has valuable hydrophilic and eudermic properties which make it useful in the preparation of emulsions (creams and milks) for pharmaceutical and cosmetic use.

The emulsions obtained by means of the product of the present invention proved to have a high stability within a wide temperature ($+4°$ - $+50°C$) and pH (3 - 10) range, and also to have an improved cutaneous tolerability.

EP 0 247 552 A2

## "SELF-EMULSIFYING FAT FOR PHARMACEUTICAL AND COSMETIC USE, PREPERATION AND USE THEREOF".

The present invention relates to a non-ionic self-emulsifying fat, consisting of a mixture of partly esterified and ethoxylated $C_6$-$C_{18}$ fatty acids and $C_6$-$C_{20}$ fatty alcohols or polyalcohols, to a process for the preparation thereof and to the use as a carrier and/or excipient in cosmetic and pharmaceutical formulations.

Non-ionic emulsifying fats are used in pharmaceutical and cosmetic technique as bases for oil/water emulsions such as creams, milks, etc. Up to now no emulsifying agents were available which fulfill at the same time the essential requirements for the intended use such as no toxicity, very good topical tolerability, long-term stability of the obtained emulsions within a wide temperature and pH range, compatibility with both acid and basic active ingredients .

The present invention allows to achieve the above cited characteristics and advantages, providing a non-ionic self-emulsifying fat prepared according to a precise operative sequence and consisting of
-$C_6$ -$C_{20}$ fatty alcohols;
-ethoxylated fatty alcohols of formula $R_1O(CH_2CH_2O)_nH$, wherein $R_1$ is an alkyl residue of a $C_6$-$C_{20}$ fatty alcohol and n is an integer from 1 to 5;
-ethoxylated fatty acids of formula $R$-COO-$(CH_2CH_2O)_nH$, wherein R is an alkyl residue of a $C_6$6-$C_{18}$ fatty acid and n is an integer from 1 to 5;
-fatty acids monoglycerides;
-optionally a polyalcohol such as glycerol.

Examples of ethoxylated and non-ethoxylated fatty alcohols, which may be comprised in the composition of the fat according to the invention, comprise hexanol, octanol, decanol, dodecanol, isotridecyl alcohol, tetradecanol, hexadecanol, octadecanol, oleyl alcohol, eicosanol.

Examples of fatty acids comprise caproic, caprylic, caprynic, lauric, myristic, palmitic, stearic, isostearic, oleic acids.

In case of ethoxylated alcohols or acids, n in the above formulae is preferably 2.

Optionally ethoxylated stearic and oleic monoglycerides are preferably used as monoglycerides.

$C_6$-$C_{20}$ fatty alcohols are present in amounts of 35-50%, ethoxylated alcohols in 5 to 15%, ethoxylated fatty acids in 15 to 25% and monoglycerides in 5 to 15%.

Fatty alcohols and acids used in the formulation of the emulsifier according to the invention show a distribution of the various molecular species which is characteristic of the commercial source from which they derive. For example, in case of fatty alcohols, $C_6$-$C_{12}$ alcohols are typically present in percentages ranging from 3 to 7%, $C_{14}$ alcohol from 0 to 2%, $C_{16}$ alcohol from 8 to 12%, $C_{18}$ and $C_{20}$ alcohols from 10 to 14%. Moreover, lesser amount of $C_{22}$ and $C_{24}$ homologues may be present.

The process for the preparation consists of two separate steps: in a first step, ethoxylated alcohols are dissolved with a part of the homologues under slow stirring and in inert atmosphere, at a temperature from 40 to 60°C. When dissolution is complete, water is removed off by distillation under vacuum.

Separately, monoglycerides are added to the fatty acids maintained at the melt state, under vacuum, at a temperature ranging from 40 to 60°C.

After removal of water by distillation under vacuum, the remaining fatty alcohols are added and the two phases are mixed under fast stirring at a temperature from 50 to 60°C.

The following example illustrates the process of the invention in more detail.

## EXAMPLE

a) A portion of the ethoxylated alcohols ($C_{12}$-$C_{14}$ alcohols 4 OE) was dissolved in a stainless steel reactor with a portion of the homologues fatty alcohols; dissolution occurred under slow stirring in a nitrogen atmosphere at a temperature from 40 to 60°C.
When the optimum temperature was attained, vacuum was applied to the reactor and water was distilled off, until no dropping was observed. The obtained mixture was transferred into a heated and thermostatized vessel, at a temperature from 50 to 60°C.

b) An exactly weighed amount of 5 OE coconut fatty acids was placed into a reactor and melted under vacuum at a temperature from 40 to 60°C; then glyceryl monooleate and monostearate 15 OE were added.
When the optimum temperature was reached, water was distilled under vacuum until no dropping was observed. Vacuum was interrupted with nitrogen and the remaining fatty alcohols were slowly added. After dissolution, the second phase was added to the first one under quick stir ring at a temperature ranging from 50 to 60°C. After 30-50 minutes the mixture was quickly cooled, under slow stirring, to a temperature of 18-22°C.

A thick creamy past, having a slight characteristic odour, was obtained.

## Characteristics of the product

Aspect: white pasty mass.
Color: varying from white to cream white.
Odour: slight and characteristic.
Solubility: soluble in oils.
Acidity index: Max 2.
Jodine index: Max 20.
Peroxide index: Max 5.
Saponification index: 80-110.
Moisture: lower than 1%.
HLB (ATLAS system): 12.1.

## Toxicological data

Acute toxicity: evaluated on Swiss albino mice weighing 25-30 g, by intraperitoneal route.
$LD_{50}$: higher than 8 g/kg.
Chronic toxicity: no toxicity.

The product caused no sensitization on epidermis and derma, after prolonged treatment on rabbit epilated cutis, neither on human cutis of healthy volunteers.

The Draize test, carried out on New Zealand rabbits at a 10% concentration, evidenced a slightly irritative action on the conjunctiva.

The emulsifier of the invention is used in the preparation of o/w emulsions, both creams and milks, in a percentage which varies from 8 to 10%; if necessary, said percentage may be increased up to 20%, obtaining the emulsion without further addition of the oily phase.

In view of its chemical nature, the fat according to the invention may conglobate active ingredients having both acid and basic characteristics, keeping stability during the time unchanged.

## Claims

CLAIMS for the Contracting States: BE, CH, DE, FR, GB, IT, LI, LU, NL, SE, AT.

1. A non-ionic emulsifying fat comprising:
-$C_6$-$C_{20}$ fatty alcohols;
-ethoxylated fatty alcohols of formula $R_1O$-$(CH_2CH_2O)_nH$, wherein $R_1$ is an alkyl residue of a $C_6$-$C_{20}$ fatty alcohol and n is an integer from 1 to 5;
-ethoxylated fatty acids of formula $R$-$COO$-$(CH_2CH_2O)_nH$, wherein R is an alkyl residue of a $C_6$-$C_{18}$ fatty acid and n is an integer from 1 to 5;
-fatty acids monoglycerides;
-optionally a polyalcohol such as glycerol.

2. A non-ionic self-emulsifying fat according to claim 1, wherein $C_6$-$C_{20}$ alcohols and $C_6$-$C_{18}$ ethoxylated acids comprise the homologues having an even number of carbon atoms.

3. A non-ionic self-emulsifying fat according to claim 1 or 2, wherein the used monoglycerides are stearic and oleic monoglycerides, which may optionally be ethoxylated.

4. A non-ionic self-emulsifying fat according to any one of the preceeding claims, wherein ethoxylated acids and alcohols, respectively of formula $R$-$COO(CH_2CH_2O)_nH$ and $R_1O(CH_2CH_2O)_nH$ have n = 2.

5. A non-ionic self-emulsifying fat according to any one of the preceeding claims, wherein $C_6$-$C_{20}$ fatty alcohols are present from 35 to 50%; ethoxylated alcohols from 5 to 15%; ethoxylated fatty acids from 15 to 25%; monoglycerides from 5 to 15%.

6. A process for the preparation of a non-ionic self-emulsifying fat as claimed in claims 1-5, which process comprises the following steps:
a) dissolution of ethoxylated alcohols in a portion of the fatty alcohols in nitrogen atmosphere and at a temperature from 40 to 60°C;
b) distillation under vacuum of the water contained in solution a);
c) addition of the monoglycerides to the fatty acids, maintained in the melted state under vacuum at a temperature from 40 to 60°C and subsequent distillation of water under vacuum;
d) addition of the remaining fatty alcohols to the mass obtained in c);
e) mixing of the phase obtained in d) to the solution obtained in b), at a temperature from 50 to 60°C, and subsequent cooling.

7. Pharmaceutical and cosmetic formulations in form of emulsions, creams or milks, in which the emulsifying agent is a non-ionic self-emulsifying fat as claimed in claims 1-5.

8. Use of a non-ionic self-emulsifying fat as claimed in claims 1-5 as a carrier and/or excipient in pharmaceutical or cosmetic formulations, in admixture with active ingredients and optionally other excipients.

CLAIM for the Contracting States: SP, GR.

1. A process for the preparation of a non-ionic self-emulsifying fat comprising:
-$C_6$-$C_{20}$ fatty alcohols;
-ethoxylated fatty alcohols of formula $R_1O$-$(CH_2CH_2O)_nH$, wherein $R_1$ is an alkyl residue of a $C_6$-$C_{20}$ fatty alcohol and n is an integer from 1 to 5;
-ethoxylated fatty acids of formula $R$-$COO$-$(CH_2CH_2O)_nH$, wherein R is an alkyl residue of a $C_6$-$C_{18}$ fatty acid and n is an integer from 1 to 5;

-fatty acids monoglycerides;

-optionally a polyalcohol such as glycerol, which process comprises the following steps:

    a) dissolution of ethoxylated alcohols in a portion of the fatty alcohols in nitrogen atmosphere and at a temperature from 40 to 60°C;

    b) distillation under vacuum of the water contained in solution a);

    c) addition of the monoglycerides to the fatty acids, maintained in the melted state under vacuum at a temperature from 40 to 60°C and subsequent distillation of water under vacuum;

    d) addition of the remaining fatty alcohols to the mass obtained in c);

    e) mixing of the phase obtained in d) to the solution obtained in b), at a temperature from 50 to 60°C, and subsequent cooling.